# EUROPEAN PATENT APPLICATION

(11) **EP 4 286 053 A1**
(43) Date of publication of application: **06.12.2023**
(21) Application number: 22745247.1
(22) Date of filing: 26.01.2022
(51) Int. Cl.: B01L 3/00

(54) **BIOLOGICAL PARTICLE SORTING FLOW CHANNEL AND MICROFLUIDIC CHIP**

(30) Priority: 29.01.2021 CN 202110130182
(71) Applicant: Guangzhou Wondfo Biotech Co., Ltd., Guangzhou, Guangdong 510663 (CN)
(72) Inventor: MENG, Xuan, Guangzhou, Guangdong 510663 (CN); YANG, Jiamin, Guangzhou, Guangdong 510663 (CN)
(74) Representative: Jacob, Reuben Ellis
(86) International application number: PCT/CN2022/073894
(87) International publication number: WO 2022/161373

(57) **Abstract**

The present invention relates to a biological particle sorting channel and a microfluidic chip. The biological particle sorting channel comprises a first channel unit, which comprises a first side wall and a second side wall disposed oppositely, wherein the first side wall and the second side wall are asymmetric curved surfaces, and the side of the first channel unit close to the first side wall or the second side wall is provided with a deepened channel in a recessed manner that is disposed in an extension direction of the first channel unit. The first channel unit having the asymmetric curved surface is provided to form a focused flow, and the deepened channel is disposed in the extension direction of the first channel unit to disrupt the flow state of the liquid in the vicinity of the first channel unit close to the deepened channel so as to change the inertial lift force and the Dean drag force and thus destroy the original equilibrium such that particles, such as white blood cells, on the side close to the deepened channel can be in a disordered motion state, preventing a band of aggregation of white blood cells from overlapping with circulating tumour cells, which is conducive to the recovery of circulating tumour cells.

## Description

### Technical Field

The present invention relates to the technical field of biological particle sorting, and in particular to a biological particle sorting channel and a microfluidic chip.

### Background Art

Inertial focusing microfluidic technology is gradually applied in cell sorting in the biomedical industry, for example, sorting circulating tumour cells from blood. Cancer ranks second among normal causes of death in the world, and tumour metastasis is the cause of 90% of cancer deaths. In the process of tumour metastasis, tumour cells are exfoliated from a primary tumour or a metastasis tumour, circulate in the lymphatic system or the peripheral blood, and then invade distant tissues and form a new tumour, eventually leading to the death of the patient. The exfoliated tumour cells are called circulating tumour cells (CTCs). The circulating tumour cells are in extremely low level, and typically, there are 1-10 circulating tumour cells per millilitre of blood, but there are millions of white blood cells and billions of red blood cells per millilitre of blood. Thus, it is difficult to sort and enrich circulating tumour cells, like finding a needle in a haystack. During a conventional linear curved channel focusing process, circulating tumour cells and white blood cells tend to overlap, which is not conducive to the sorting of circulating tumour cells.

### Summary

In view of this, aiming at the above problems, it is necessary to provide a biological particle sorting channel and a microfluidic chip, which can effectively prevent overlapping of circulating tumour cells and white blood cells to facilitate sorting of circulating tumour cells.

A biological particle sorting channel comprises a first channel unit, which comprises a first side wall and a second side wall disposed oppositely, wherein the first side wall and the second side wall are asymmetric curved surfaces, and the side of the first channel unit close to the first side wall or the second side wall is provided with a deepened channel in a recessed manner that is disposed in an extension direction of the first channel unit.

When a sample, such as blood, is passed into the biological particle sorting channel for sorting of particles, by means of providing the first channel unit having asymmetric curved surfaces, due to the influence of inertial lift force, Dean drag force, etc., the particles in the sample, such as white blood cells and circulating tumour cells in the blood, move relative to each other in the cross-section of the first channel unit, and when each type of particles move to an equilibrium position in the cross-section, the particles are stabilized at this position in the cross-section, so that the particles are focused at a stable position in the cross-section to form a focused flow that flows downstream. The particles, such as white blood cells and circulating tumour cells, in blood have different diameters and are accumulated at different positions in the cross-section of the first channel unit, but the band of inertial accumulation of certain particles such as the white blood cells is in close proximity to the band of inertial accumulation of the circulating tumour cells. The side of the first channel unit close to the first side wall or the second side wall (e.g., the side away from the accumulation of the circulating tumour cells) is provided with the deepened channel in a recessed manner that is disposed in the extension direction of the first channel unit, the flow state of the liquid in the vicinity of the first channel unit close to the deepened channel is disrupted so as to change the inertial lift force and the Dean drag force and thus destroy the original equilibrium, so that the particles, such as the white blood cells, on the side close to the deepened channel can be in a disordered motion state, preventing the band of aggregation of the white blood cells from overlapping with the circulating tumour cells, which not only ensures that the aggregation of circulating tumour cells is not disturbed, but also prevents the white blood cells from accumulating on the same side as the circulating tumour cells, which is convenient for the subsequent separation of the circulating tumour cells and the white blood cells and is conducive to the recovery of the circulating tumour cells.

In one of the embodiments, the biological particle sorting channel further comprises a second channel unit that is in communication with the first channel unit, wherein the radius of curvature of the second channel unit is less than that of the first channel unit, the deepened channel correspondingly extends into the second channel unit, and the second channel unit comprises a third side wall and a fourth side wall disposed oppositely, the third side wall and the fourth side wall being asymmetric curved surfaces, the third side wall being connected to the second side wall, and the fourth side wall being connected to the first side wall.

In one of the embodiments, the biological particle sorting channel comprises a plurality of first channel units and a plurality of second channel units disposed alternately.

In one of the embodiments, the biological particle sorting channel further comprises a third channel unit that is in communication with the second channel unit at the tail, wherein the radius of curvature of the third channel unit is greater than that of the first channel unit, the deepened channel correspondingly extends into the third channel unit, the third channel unit is rotated by 90° relative to the first channel unit, and the third channel unit comprises a fifth side wall and a sixth side wall disposed oppositely, the fifth side wall and the sixth side wall being asymmetric curved surfaces, the fifth side wall being connected to the end of the third side wall away from the second side wall, and the sixth side wall being connected to the end of the fourth side wall away from the first side wall.

In one of the embodiments, the biological particle sorting channel further comprises a plurality of fourth channel units and a plurality of fifth channel units, wherein the radius of curvature of the fourth channel unit is less than that of the fifth channel unit, the radius of curvature of the third channel unit is greater than that of the fifth channel unit, the fourth channel unit and the fifth channel unit are disposed alternately, and the deepened channel correspondingly extends into the fourth channel unit and the fifth channel unit; and the third channel unit is rotated by 90° relative to the fifth channel unit, and the fourth channel unit comprises a seventh side wall and an eighth side wall which are disposed oppositely and are asymmetric curved surfaces, and the fifth channel unit comprises a ninth side wall and a tenth side wall which are disposed oppositely and are asymmetric curved surfaces, the eighth side wall being connected to the ninth side wall, the seventh side wall being connected to the tenth side wall, the seventh side wall of the fourth channel unit at the head being connected to the end of the fifth side wall away from the third side wall, the eighth side wall of the fourth channel unit at the head being connected to the end of the sixth side wall away from the fourth side wall, and at least one of the fifth channel units being provided with a flow dividing hole penetrating a wall surface of the fifth channel unit.

In one of the embodiments, the fifth channel unit is provided with a blocking member corresponding to an inlet of the flow dividing hole, the blocking member being located on the side of the flow dividing hole away from the deepened channel, and the width of the blocking member in an extension direction of the fifth channel unit being greater than the diameter of the flow dividing hole.

In one of the embodiments, the biological particle sorting channel further comprises a sixth channel unit that is in communication with the fourth channel unit at the tail, wherein the radius of curvature of the sixth channel unit is greater than that of the fifth channel unit, the deepened channel correspondingly extends into the sixth channel unit, the sixth channel unit is rotated by 90° relative to the fifth channel unit, the sixth channel unit protrudes in the opposite direction of the third channel unit, and the sixth channel unit comprises an eleventh side wall and a twelfth side wall disposed oppositely, the eleventh side wall and the twelfth side wall being asymmetric curved surfaces, the eleventh side wall being connected to the end of the eighth side wall away from the ninth side wall, and the twelfth side wall being connected to the end of the seventh side wall away from the tenth side wall.

In one of the embodiments, the deepened channel has a depth of 50 µm to 200 µm; or the deepened channel has a depth of 70 µm to 120 µm.

In one of the embodiments, the radius of curvature of the first side wall is greater than or less than that of the second side wall, and the first side wall and the second side wall protrude toward the same side;
and/or, the radius of curvature of the third side wall is less than that of the fourth side wall, and the third side wall and the fourth side wall protrude toward the same side;
and/or, the radius of curvature of the fifth side wall is less than that of the sixth side wall, and the fifth side wall and the sixth side wall protrude toward the same side;
and/or, the radius of curvature of the seventh side wall is less than that of the eighth side wall, and the seventh side wall and the eighth side wall protrude toward the same side;
and/or, the radius of curvature of the ninth side wall is less than that of the tenth side wall, and the ninth side wall and the tenth side wall protrude toward the same side;
and/or, the radius of curvature of the eleventh side wall is less than that of the twelfth side wall, and the eleventh side wall and the twelfth side wall protrude toward the same side.

A microfluidic chip comprises a functional board, wherein a first side surface of the functional board is provided with the biological particle sorting channel.

A microfluidic chip comprises a functional board, wherein a first side surface of the functional board is provided with the biological particle sorting channel, and a second side surface of the functional board is provided with a buffer channel of a repeatedly folded structure, the buffer channel being in communication with the flow dividing hole.

### Brief Description of the Drawings

FIG. 1 is a schematic diagram of a biological particle sorting channel according to an embodiment;
FIG. 2 is a schematic diagram of a first side surface of a functional board of a microfluidic chip according to an embodiment;
FIG. 3 is a schematic diagram of a second side surface of the functional board of a microfluidic chip according to an embodiment;
FIG. 4 is a schematic cross-sectional view of the part in FIG. 2 along section line A-A;
FIG. 5 is a schematic diagram of an upper cover plate of the microfluidic chip according to an embodiment; and
FIG. 6 is a schematic diagram of a lower cover plate of the microfluidic chip according to an embodiment.

### List of reference signs:

01. red blood cell; 02. white blood cell; 03. circulating tumour cell; 1. biological particle sorting channel; 10. first channel unit; 110. first side wall; 120. second side wall; 2. deepened channel; 20. second channel unit; 210. third side wall; 220. fourth side wall; 30. third channel unit; 310. fifth side wall; 320. sixth side wall; 40. fourth channel unit; 410. seventh side wall; 420. eighth side wall; 50. fifth channel unit; 510. ninth side wall; 520. tenth side wall; 51. flow dividing hole; 52. blocking member; 60. sixth channel unit; 610. eleventh side wall; 620. twelfth side wall; 70. first joining unit; 710. thirteenth side wall; 720. fourteenth side wall; 80. second joining unit; 810. fifteenth side wall; 820. sixteenth side wall; 3. buffer channel; 4. inlet channel; 5. recovery channel; 501. first outflow hole; 6. waste liquid channel; 601. second outflow hole; 11. functional board; 101. sample injection hole; 22. upper cover plate; 201. sample inlet; 33. lower cover plate; 301. recovery hole; 302. waste liquid hole; 303. discharge hole.

### Detailed Description of the Preferred Embodiments

In order to make the above objectives, features, and advantages of the present invention more apparent, the particular implementations of the present invention will be described in detail below in conjunction with the accompanying drawings. In the following description, numerous specific details are set forth in order to fully understand the present invention. However, the present invention can be implemented in many other ways different from those described herein, and those skilled in the art can make similar improvements without departing from the essence of the present invention. Therefore, the present invention is not limited by the particular embodiments disclosed below.

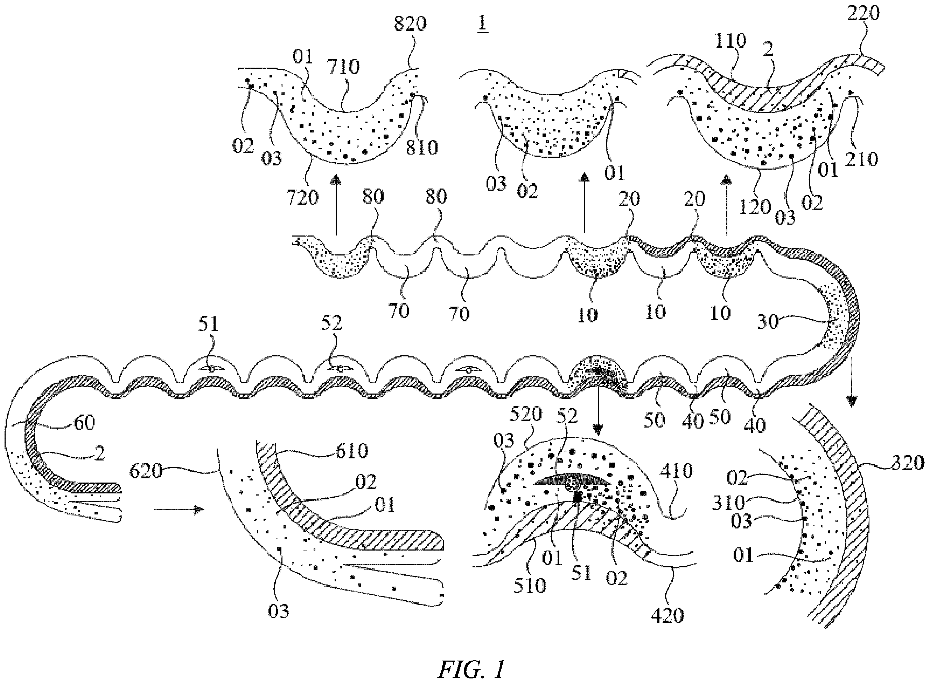

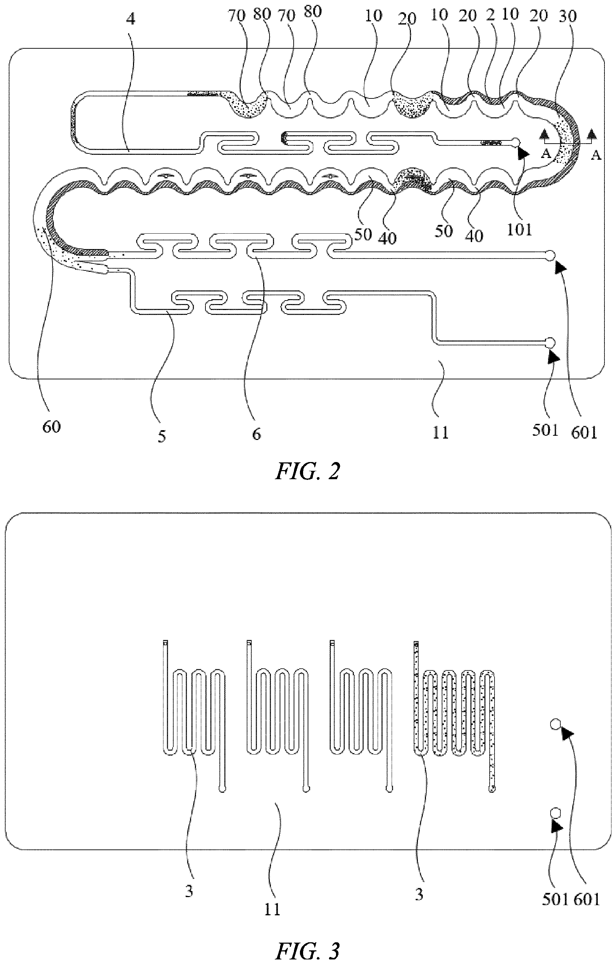

Referring to FIGS. 1 and 2, according to an embodiment, a microfluidic chip is provided, comprising a functional board 11, with a first side surface of the functional board 11 being provided with a biological particle sorting channel 1.

Referring to FIG. 1, in one of the embodiments, the biological particle sorting channel 1 comprises a first channel unit 10. The first channel unit 10 comprises a first side wall 110 and a second side wall 120 disposed oppositely, the first side wall 110 and the second side wall 120 are asymmetric curved surfaces, the side of the first channel unit 10 close to first side wall 110 or the second side wall 120 is provided with a deepened channel 2 in a recessed manner, and the deepened channel 2 is disposed in an extension direction of the first channel unit 10.

FIG. 1 is a schematic structural diagram of the biological particle sorting channel 1, showing schematic enlarged views of motion states of circulating tumour cells, white blood cells and red blood cells in different channel units of the biological particle sorting channel 1. When a sample, such as blood, is passed into the biological particle sorting channel 1 for sorting of particles, by means of providing the first channel unit 10 having asymmetric curved surfaces, due to the influence of inertial lift force, Dean drag force, etc., the particles in the sample, such as white blood cells 02 and circulating tumour cells 03 in the blood, move relative to each other in the cross-section of the first channel unit 10, and when each type of particles move to an equilibrium position in the cross-section, the particles are stabilized at this position in the cross-section, so that the particles are focused at a stable position in the cross-section to form a focused flow that flows downstream. The particles, such as white blood cells 02 and circulating tumour cells 03, in blood have different diameters and are accumulated at different positions in the cross-section of the first channel unit 10, but the band of inertial accumulation of certain particles such as the white blood cells 02 is in close proximity to the band of inertial accumulation of the circulating tumour cells 03. The side of the first channel unit 10 close to the first side wall 110 or the second side wall 120 (e.g., the side away from the accumulation of the circulating tumour cells 03) is provided with the deepened channel 2 in a recessed manner, the deepened channel 2 being disposed in the extension direction of the first channel unit 10, the flow state of the liquid in the vicinity of the first channel unit 10 close to the deepened channel 2 is disrupted so as to change the inertial lift force and the Dean drag force and thus destroy the original equilibrium such that the particles, such as the white blood cells 02, on the side close to the deepened channel 2 can be in a disordered motion state, preventing a band of aggregation of the white blood cells 02 from overlapping with the circulating tumour cells 03, which not only ensures that the aggregation of the circulating tumour cells 03 is not disturbed, but also prevents the white blood cells 02 from accumulating on the same side as the circulating tumour cells 03, which is convenient for the subsequent separation of the circulating tumour cells 03 and the white blood cells 02 and is conducive to the recovery of the circulating tumour cells 03.

In one of the embodiments, the biological particle sorting channel 1 further comprises a second channel unit 20 that is in communication with the first channel unit 10, the radius of curvature of the second channel unit 20 is less than that of the first channel unit 10, the deepened channel 2 correspondingly extends into the second channel unit 20, and the second channel unit 20 comprises a third side wall 210 and a fourth side wall 220 disposed oppositely, the third side wall 210 and the fourth side wall 220 being asymmetric curved surfaces, the third side wall 210 being connected to the second side wall 120, and the fourth side wall 220 being connected to the first side wall 110. The first channel unit 10 is connected to the second channel unit 20 that has a smaller radius of curvature than the first channel unit 10, and the deepened channel 2 correspondingly extends into the second channel unit 20 so as to further adjust the focusing and equilibrium position of the particles, so that under the combined action of the first channel unit 10 having a larger radius of curvature and the second channel unit 20 having a smaller radius of curvature, the particles accumulate to form a band at the equilibrium position in the cross-section of the biological particle sorting channel 1.

Referring to FIGS. 1 and 2, further, the biological particle sorting channel 1 comprises a plurality of first channel units 10 and a plurality of second channel units 20. The first channel units 10 and the second channel units 20 are disposed alternately. The plurality of first channel units 10 and the plurality of the second channel units 20 are connected alternately to form a wave-shaped channel to exert a physical action on the particles, which is conducive to reducing the length of the channel and the effect of forming a band by inertial focusing.

Referring to FIGS. 1 and 2, in one of the embodiments, the biological particle sorting channel 1 further comprises a third channel unit 30 that is in communication with the second channel unit 20 at the tail, the radius of curvature of the third channel unit 30 is greater than that of the first channel unit 10, the deepened channel 2 correspondingly extends into the third channel unit 30, the third channel unit 30 is rotated by 90° relative to the first channel unit 10, and the third channel unit 30 comprises a fifth side wall 310 and a sixth side wall 320 disposed oppositely, the fifth side wall 310 and the sixth side wall 320 being asymmetric curved surfaces, the fifth side wall 310 being connected to the end of the third side wall 210 away from second side wall 120, and the sixth side wall 320 being connected to the end of the fourth side wall 220 away from first side wall 110. The deepened channel 2 correspondingly extends into the third channel unit 30, and under the action of the deepened channel, the white blood cells 02 are distributed more evenly in the third channel unit 30 and prevented from overlapping with the circulating tumour cells 03. The second channel unit 20 at the tail is in communication with the third channel unit 30 that is rotated by 90°. Since the third channel unit 30 has a larger radius, the relative advantage of the inertial lift force is even greater. When passing through the third channel unit 30, the fluid in the middle of the channel receives the largest centrifugal force and thus flows to outer edge of the channel. The fluid has the smallest flow velocity near the channel wall and also receives the smallest centrifugal force, and is thus compressed by the fluid in the middle. In this way, under the action of the third channel unit 30, the circulating tumour cells 03 can be as close as possible to the bottom of the inner wall of the pipe and are ready for the subsequent sorting of the circulating tumour cells 03 and removal of the white blood cells 02.

Further, referring to FIGS. 1 and 2, in one of the embodiments, the biological particle sorting channel 1 further comprises a plurality of fourth channel units 40 and a plurality of fifth channel units 50, the radius of curvature of the fourth channel unit 40 is less than that of the fifth channel unit 50, the radius of curvature of the third channel unit 30 is greater than that of the fifth channel unit 50, the fourth channel unit 40 and the fifth channel unit 50 are disposed alternately, and the deepened channel 2 correspondingly extends into the fourth channel unit 40 and the fifth channel unit 50. The third channel unit 30 is rotated by 90° relative to the fifth channel unit 50. The fourth channel unit 40 comprises a seventh side wall 410 and an eighth side wall 420 disposed oppositely, the seventh side wall 410 and the eighth side wall 420 being asymmetric curved surfaces. The fifth channel unit 50 comprises a ninth side wall 510 and a tenth side wall 520 disposed oppositely, the ninth side wall 510 and the tenth side wall 520 being asymmetric curved surfaces. The eighth side wall 420 is connected to the ninth side wall 510, the seventh side wall 410 is connected to the tenth side wall 520, the seventh side wall 410 of the fourth channel unit 40 at the head is connected to the end of the fifth side wall 310 away from the third side wall 210, the eighth side wall 420 of the fourth channel unit 40 at the head is connected to the end of the sixth side wall 320 away from the fourth side wall 220, and at least one of the fifth channel units 50 is provided with a flow dividing hole 51 penetrating a wall surface of the fifth channel unit. The sample sequentially flows from the third channel unit 30 into the fourth channel units 40 and the fifth channel units 50 which are disposed alternately, the fourth channel unit 40, the fifth channel unit 50 and the third channel unit 30 are disposed at 90°, and thus the fourth channel units 40 and the fifth channel units 50 are connected alternately to form a wave-shaped channel for further sorting of the particles, which is conducive to reducing the length of the channel and the effect of forming a band by inertial focusing. At least one of the fifth channel units 50 is provided with the flow dividing hole 51 penetrating the wall surface of the fifth channel unit, so that the particles having larger diameters, such as circulating tumour cells 03, continue to move against the bottom of the inner wall of the fifth channel unit 50 (on the side away from the deepened channel 2). Since the particles having smaller diameters, such as the white blood cells 02 and the red blood cells 01, are distributed more evenly in the channel, some of the white blood cells 02 and the red blood cells 01 flow out of the flow dividing hole 51, which is conducive to the subsequent recovery of the circulating tumour cells 03.

Moreover, as the liquid flows out from the flow dividing hole 51, the flow velocity of the liquid in the channel becomes relatively lower, the circulating tumour cells 03 will change slightly in movement trajectory and then easily move away from the inner wall of the channel, and thus the circulating tumour cells 03 easily approach the flow dividing hole 51, so that by means of providing the third channel unit 30 having a larger radius of curvature at the position where the fourth channel unit 40 is joined with the fifth channel unit 50, the circulating tumour cells 03 flow against the bottom of the inner wall of the channel after flowing through the large-bend third channel unit 30, preventing the circulating tumour cells 03 from flowing into the flow dividing hole 51, thereby improving the recovery rate of the circulating tumour cells 03.

Referring to FIGS. 1 and 2, in one of the embodiments, the fifth channel unit 50 is provided with a blocking member 52 corresponding to an inlet of the flow dividing hole 51, the blocking member 52 is located on the side of the flow dividing hole 51 away from the deepened channel 2, and the width of the blocking member 52 in an extension direction of the fifth channel unit 50 is greater than the diameter of the flow dividing hole 51. Providing the blocking member 52 on the side away from the deepened channel 2 prevents the particles, such as circulating tumour cells 03, focused on this side from flowing into the flow dividing hole 51, and the width of the blocking member 52 in the extension direction of the fifth channel unit 50 is greater than the diameter of the flow dividing hole 51, so that the particles focused on the side away from the deepened channel 2 in the flowing direction of the particles are blocked by the blocking member 52 for a long distance, while the white blood cells 02 and the red blood cells 01 flow out from the flow dividing hole 51 due to even distribution of the white blood cells 02 and the red blood cells 01 in the channel.

Referring to FIG. 3, in one of the embodiments, a second side surface of the functional board 11 is provided with a buffer channel 3 that is in communication with the flow dividing hole 51, and the buffer channel 3 is of a repeatedly folded structure to allow the outflowing liquid to be in a stable state, so as to prevent the flow of the liquid remaining in the biological particle sorting channel 1 from being disturbed by shaking and thus affecting the subsequent recovery of the circulating tumour cells 03.

Referring to FIGS. 1 and 2, further, in one of the embodiments, the biological particle sorting channel 1 further comprises a sixth channel unit 60 that is in communication with the fourth channel unit 40 at the tail, the radius of curvature of the sixth channel unit 60 is greater than that of the fifth channel unit 50, the deepened channel 2 correspondingly extends into the sixth channel unit 60, and the sixth channel unit 60 is rotated by 90° relative to the fifth channel unit 50. The sixth channel unit 60 protrudes in the opposite direction of the third channel unit 30. The sixth channel unit 60 comprises an eleventh side wall 610 and a twelfth side wall 620 disposed oppositely, the eleventh side wall 610 and the twelfth side wall 620 are asymmetric curved surfaces, the eleventh side wall 610 is connected to the end of the eighth side wall 420 away from the ninth side wall 510, and the twelfth side wall 620 is connected to the end of the seventh side wall 520 away from the tenth side wall 410. After the blood sample flows through the flow dividing hole 51, the contents of the white blood cells 02 and the red blood cells 01 gradually decrease, and the flow velocities thereof also gradually decrease, so that by means of joining with the sixth channel unit 60 that has a larger radius of curvature than the fourth channel unit 40 and the fifth channel unit 50, the movement trajectory of the circulating tumour cells 03 can be stabilized, which is convenient for the subsequent recovery of the circulating tumour cells 03.

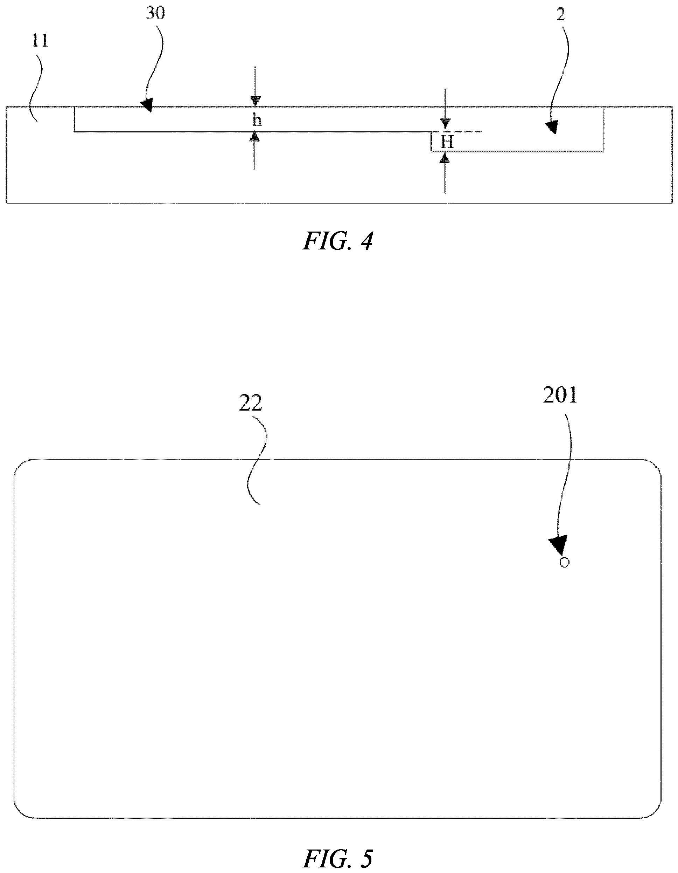

Referring to FIG. 4, optionally, the biological particle sorting channel 1 has a depth h of 100 µm to 200 µm relative to the first side surface of the functional board. Preferably, the biological particle sorting channel 1 has a depth h of 110 µm to 150 µm relative to the first side surface of the functional board. In one of the embodiments, the deepened channel 2 has a depth H of 50 µm to 200 µm, that is, the deepened channel 2 is 50 µm to 200 µm deeper than the biological particle sorting channel 1. Preferably, the deepened channel 2 has a depth of 70 µm to 120 µm, that is, the deepened channel 2 is 70 µm to 120 µm deeper than the biological particle sorting channel 1. It is possible to not only ensure that the circulating tumour cells 03 can flow as close as possible to the bottom of the inner wall of the channel in each channel unit of the biological particle sorting channel 1, but also disturb the liquid flow state on one side of the deepened channel 2, which prevents the band of aggregation of the white blood cells 02 from overlapping with the circulating tumour cells 03 and thus affecting the subsequent recovery rate and purity of the circulating tumour cells 03.

Referring to FIG. 2, in one of the embodiments, one end of the sixth channel unit 60 is provided with a recovery channel 5 and a waste liquid channel 6 independent of each other, the recovery channel 5 being in communication with the side of the sixth channel unit 60 away from the deepened channel 2 (the side where the circulating tumour cells 03 focus), and the waste liquid channel 6 being in communication with the side of the sixth channel unit 60 close to the deepened channel 2. The circulating tumour cells 03 flow against the inner wall of the sixth channel unit 60 and into the recovery channel 5, while the white blood cells 02 and the red blood cells 01 flow into the waste liquid channel 6. In one of the embodiments, the waste liquid channel 6 and the recovery channel 5 are of a repeatedly folded structure to stabilize the liquid flow state, preventing the movement trajectory of the circulating tumour cells 03 at the tail of the biological particle sorting channel 1 from being affected during the subsequent outflow and drop of the liquid.

Referring to FIGS. 1 and 2, further, in one of the embodiments, the biological particle sorting channel 1 further comprises a first joining unit 70 and a second joining unit 80 connected alternately, the radius of curvature of the first joining unit 70 being greater than that of the second joining unit 80. The first joining unit 70 comprises a thirteenth side wall 710 and a fourteenth side wall 720 disposed oppositely, the thirteenth side wall 710 and the fourteenth side wall 720 being asymmetric curved surfaces. The second joining unit 80 comprises a fifteenth side wall 810 and a sixteenth side wall 820 disposed oppositely, the fifteenth side wall 810 and the sixteenth side wall 820 being asymmetric curved surfaces. The fourteenth side wall 720 is connected to the fifteenth side wall 810, the thirteenth side wall 710 is connected to the sixteenth side wall 820, the first side wall 110 of the first channel unit 10 at the head is connected to the sixteenth side wall 820 of the second joining unit 80 at the tail, and the second side wall 120 of the first channel unit 10 at the head is connected to the fifteenth side wall 810 of the second joining unit 80 at the tail. The first joining unit 70 and the second joining unit 80 are connected alternately to form a wave-shaped channel, the width of the first joining unit 70 perpendicular to the extension direction thereof being less than that of the first channel unit 10 perpendicular to the extension direction thereof. In order to avoid the situation that the width of the channel suddenly becomes larger when the blood sample enters the biological particle sorting channel 1 from an inlet channel 4, which disturbs the original movement trajectory of the circulating tumour cells 03, to prevent destroying the trajectory of the circulating tumour cells 03 moving against the inner wall, the transition between the first joining unit 70, which has a smaller width than the first channel unit 10, and the second joining unit 80 is provided to achieve a buffering effect to avoid the situation that the circulating tumour cells 03 need to flow through more wave-shaped first channel units 10 and second channel units 20 before accumulating into a thin band close to the inner wall of the channel, and the length of the biological particle sorting channel 1 can also be shortened.

Referring to FIG. 1, optionally, the first channel unit 10 and the second channel unit 20 which are disposed close to the first joining unit 70 may be provided with no deepened channel 2, so that the sample first flows stably from the first joining unit 70 and the second joining unit 80 into the first channel unit 10 and the second channel unit 20 which are provided with no deepened channel 2, so as to avoid disturbing the focus trajectory of the circulating tumour cells 03. The first channel unit 10 and the second channel unit 20, which are disposed at the end away from the first joining unit 70, are provided with the deepened channel 2, and then the circulating tumour cells 03 are located at the inner bottom of the channel, while the white blood cells 02 have not yet focused on the bottom of the channel, and the deepened channel 2 is provided to disturb the focus trajectory of the white blood cells 02, so that the white blood cells 02 move toward the side where the deepened channel 2 is located and thus avoid overlapping with the circulating tumour cells 03.

Referring to FIG. 2, further, in one of the embodiments, the first side surface of the functional board 11 is further provided with the inlet channel 4, with one end of the inlet channel 4 being provided with a sample injection hole 101, and the other end thereof being connected to the first joining unit 70 of the biological particle sorting channel 1. The inlet channel 4 is of a repeatedly folded structure. When the sample enters the inlet end of the inlet channel 4, the red blood cells 01, the white blood cells 02 and the circulating tumour cells 03 in the sample are evenly distributed in the channel. The red blood cells 01 have diameters of about 6-8 µm, the white blood cells 02 have diameters of about 8-12 µm, and the circulating tumour cells 03 have diameters of about 20-30 µm. When the liquid flows through several bends of the inlet channel 4, the white blood cells 02 and the circulating tumour cells 03 slowly accumulate, while the red blood cells 01 are still evenly distributed. The longer the inlet channel 4 that the cells flow through, the thinner the bands of the white blood cells 02 and the circulating tumour cells 03 accumulate. When the liquid slows into the biological particle sorting channel 1, the white blood cells 02 and the circulating tumour cells 03 accumulate into thinner bands and are close to the inner wall of the inlet channel 4. The inlet channel 4 is configured to be of a repeatedly folded structure, which can not only have a buffering effect on the inflowing blood sample, but also make the liquid flow state more stable, which is convenient for the circulating tumour cells 03 and the white blood cells 02 to accumulate into bands in the inlet channel 4.

Compared with the biological particle sorting channel 1, the inlet channel 4 is an elongated channel. Optionally, the inlet channel 4 has a width of 0.3 mm to 1.2 mm. Preferably, the inlet channel 4 has a width of 0.5 mm to 0.9 mm. Optionally, the inlet channel 4 has a depth of 0.06 mm to 0.3 mm. Preferably, the inlet channel 4 has a depth of 0.1 mm to 0.2 mm. With such an arrangement, the flow velocity of the blood sample that enters the inlet channel 4 tends to be stable, and the circulating tumour cells 03 and the white blood cells 02 can also be initially accumulated into bands, which is convenient for the subsequent sorting of the circulating tumour cells 03. In other embodiments, in order to achieve the purpose of buffering, the inlet channel 4 may also be formed in a repeatedly folded "S" or spiral shape.

Referring to FIG. 1, optionally, the radius of curvature of the first side wall 110 is less than or greater than that of the second side wall 120, and the first side wall 110 and second side wall 120 protrude toward the same side. In this way, the first channel unit 10 forms an asymmetric curved channel. In one of the embodiments, the radius of curvature of the first side wall 110 is greater than that of the second side wall 120, and the radius of curvature of the thirteenth side wall 710 of the first joining unit 70 is less than that of the fourteenth side wall 720, so that the width of the first joining unit 70 perpendicular to the extension direction thereof is less than that of the first channel unit 10 perpendicular to the extension direction thereof.

In one of the embodiments, the radius of curvature of the third side wall 210 is less than that of the fourth side wall 220, and the third side wall 210 and the fourth side wall 220 protrude toward the same side. In this way, the second channel unit 20 forms an asymmetric curved channel, which achieves connection between the adjacent first channel units 10 and also achieves better sorting of particles.

In one of the embodiments, the radius of curvature of the fifth side wall 310 is less than that of the sixth side wall 320, and the fifth side wall 310 and the sixth side wall 320 protrude toward the same side. In this way, the third channel unit 30 forms an asymmetric curved channel, and when the biological particle sorting channel 1 is rotated by 90°, the circulating tumour cells 03 are closer to the inner bottom of the channel.

In one of the embodiments, the radius of curvature of the seventh side wall 410 is less than that of the eighth side wall 420, and the seventh side wall 410 and the eighth side wall 420 protrude toward the same side. In this way, the fourth channel unit 40 forms an asymmetric curved channel, which achieves connection between the adjacent fifth channel units 50 and also achieves better sorting of particles.

In one of the embodiments, the radius of curvature of the ninth side wall 510 is less than or greater than that of the tenth side wall 520, and the ninth side wall 510 and the tenth side wall 520 protrude toward the same side. In this way, the fifth channel unit 50 forms an asymmetric curved channel, which achieves better sorting of particles and is convenient for the circulating tumour cells 03 to focus on one side of the tenth side wall 520.

In one of the embodiments, the radius of curvature of the eleventh side wall 610 is less than that of the twelfth side wall 620, and the eleventh side wall 610 and the twelfth side wall 620 protrude toward the same side. In this way, the sixth channel unit 60 forms an asymmetric curved channel, and when the biological particle sorting channel 1 is rotated by 90° again, the circulating tumour cells 03 are closer to the inner bottom of the channel, which is convenient for the subsequent recovery of the circulating tumour cells 03 on the side close to the twelfth side wall 620.

The biological particle sorting channel 1 is an asymmetric curved channel as a whole. When the fluid flows in the curved channel, the fluid flowing in a parabola has the highest velocity in the middle of the channel. When passing through the turning parts of the channel, the fluid in the middle of the microchannel receives the largest centrifugal force because of its maximum flow velocity, and then flows to the outer side wall of the curved channel. The fluid has the smallest flow velocity near the channel wall and also receives the smallest centrifugal force, and is thus compressed by the fluid having the high flow velocity in the middle. In order to keep the mass conservation everywhere in the fluid, in the direction perpendicular to the fluid flow, a pair of counter-rotating and symmetrical vortices are formed and are respectively located at the upper and lower parts in the cross-section of the channel, thereby generating a secondary flow of the Dean vortex. The Dean vortices will have a drag action on the particles in the fluid, which is called Dean drag force. In the curved channel, the flowing particles are under the action of both the inertial lift force and the Dean drag force, and the relative magnitude of the two forces determines the focused flow of the particles flowing in the curved channel. In this embodiment, in the biological particle sorting channel 1, the circulating tumour cells 03 are focused into a band on the inner wall of the channel under the action of the inertial lift force and the Dean drag force.

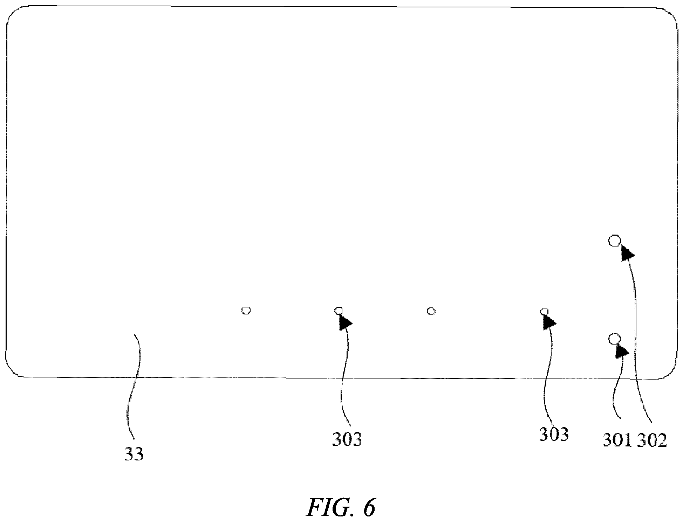

Referring to FIGS. 2-6, in one of the embodiments, the microfluidic chip further comprises an upper cover plate 22 and a lower cover plate 33. The upper cover plate 22 is bonded to the first side surface of the functional board 11, and the lower cover plate 33 is bonded to the second side surface of the functional board 11, so that the channels on the first side surface and the second side surface of the functional board 11 form a sealed channel. The upper cover plate 22 is provided with a sample inlet 201 that is in communication with the sample injection hole 101 of the inlet channel 4 of the first side surface of the functional board 11, so that the blood sample can be introduced into the microfluidic chip from the sample inlet 201. The lower cover plate 33 is provided with a recovery hole 301, a waste liquid hole 302 and a discharge hole 303. The flow dividing hole 51 is in communication with the discharge hole 303, and some of the white blood cells 02 and the red blood cells 01 flow to the second side surface of the functional board 11 via the flow dividing hole 51 and flow out of the microfluidic chip via the discharge hole 303 of the lower cover plate 33. The recovery channel 5 is in communication with the recovery hole 301 via the first outflow hole 501 that penetrates the first side surface and the second side surface of the functional board 11, and the sorted circulating tumour cells 03 flows out of the microfluidic chip via the recovery hole 301 of the lower cover plate 33. The waste liquid channel 6 is in communication with the waste liquid hole 302 via the second outflow hole 601 that penetrates the first side surface and the second side surface of the functional board 11, and the remaining liquid flows out of the microfluidic chip via the waste liquid hole 302 of the lower cover plate 33.

In the description of the present invention, it should be understood that orientation or position relationships indicated by terms such as "centre", "longitudinal", "transverse", "length", "width", "thickness", "up", "down", "front", "rear", "left", "right", "vertical", "horizontal", "top", "bottom", "inside", "outside", "clockwise", "counterclockwise", "axial", "radial", and "circumferential" are based on orientation or position relationships shown in the accompanying drawings and are merely for ease of description of the present invention and simplification of the description, rather than indicating or implying that the apparatuses or elements referred to must have a specific orientation or be constructed and operated in a specific orientation, and therefore cannot be construed as limiting the present invention.

In addition, the terms "first" and "second" are used for descriptive purposes only and cannot be construed as indicating or implying relative importance or implicitly indicating the number of technical features indicated. Therefore, the features defined with "first" and "second" may explicitly or implicitly include at least one of the features. In the description of the present invention, the phrase "a plurality of" means at least two, such as two, three, etc., unless otherwise specifically defined.

In the present invention, unless explicitly specified or defined otherwise, the terms such as "mounting", "connection", "connected" and "fixing" should be interpreted in a broad sense, for example, may be a fixed connection, a detachable connection, or integration; or may be a mechanical connection or an electrical connection; and may be a direct connection or an indirect connection via an intermediate medium, or may be communication between interiors of two elements or interaction between the two elements, unless otherwise specifically defined. For those of ordinary skill in the art, the specific meaning of the terms mentioned above in the present invention should be construed according to specific circumstances.

In the present invention, unless otherwise explicitly specified and defined, the expression of a first feature being "on" or "under" a second feature may be the case that the first feature is in direct contact with the second feature, or the first feature is in indirect contact with the second feature via an intermediate medium. Moreover, the expression of the first feature being "over", "above" and "on top of" the second feature may be the case that the first feature is directly above or obliquely above the second feature, or only means that the level of the first feature is higher than the second feature. The expression of the first feature being "underneath", "below" and "beneath" the second feature may be the case that the first feature is directly below or obliquely below the second feature, or only means that the level of the first feature is lower than the second feature.

It should be noted that when an element is referred to as being "fixed to" or "disposed on" a further element, it may be directly on the further element, or there may be an intermediate element. When one element is considered to be "connected" to a further element, the element may be directly connected to the further element, or an intermediate element may exist simultaneously. The terms "vertical", "horizontal", "upper", "lower", "left", "right" and similar expressions used herein are for illustrative purposes only but do not represent any unique implementation.

The technical features of the above-described embodiments may be combined arbitrarily. For the purpose of simplicity in description, all the possible combinations of the technical features in the above-described embodiments are not described. However, as long as there is no contradiction among the combinations of these technical features, they shall all fall within the scope of the specification.

The above-mentioned embodiments merely represent several examples of the present invention, giving specifics and details thereof, but should not be understood as limiting the scope of the present patent of invention thereby. It should be noted that a person of ordinary skill in the art could also make several alterations and improvements without departing from the spirit of the present invention and these would all fall within the scope of protection of the present invention. Therefore, the scope of protection of the present patent of invention shall be in accordance with the appended claims.

## Claims

1. A biological particle sorting channel, **characterized by** comprising a first channel unit, which comprises a first side wall and a second side wall disposed oppositely, wherein the first side wall and the second side wall are asymmetric curved surfaces, and the side of the first channel unit close to the first side wall or the second side wall is provided with a deepened channel in a recessed manner that is disposed in an extension direction of the first channel unit.

2. The biological particle sorting channel according to claim 1, **characterized by** further comprising a second channel unit that is in communication with the first channel unit, wherein the radius of curvature of the second channel unit is less than that of the first channel unit, the deepened channel correspondingly extends into the second channel unit, and the second channel unit comprises a third side wall and a fourth side wall disposed oppositely, the third side wall and the fourth side wall being asymmetric curved surfaces, the third side wall being connected to the second side wall, and the fourth side wall being connected to the first side wall.

3. The biological particle sorting channel according to claim 2, **characterized by** comprising a plurality of first channel units and a plurality of second channel units disposed alternately.

4. The biological particle sorting channel according to claim 3, **characterized by** further comprising a third channel unit that is in communication with the second channel unit at the tail, wherein the radius of curvature of the third channel unit is greater than that of the first channel unit, the deepened channel correspondingly extends into the third channel unit, the third channel unit is rotated by 90° relative to the first channel unit, and the third channel unit comprises a fifth side wall and a sixth side wall disposed oppositely, the fifth side wall and the sixth side wall being asymmetric curved surfaces, the fifth side wall being connected to the end of the third side wall away from the second side wall, and the sixth side wall being connected to the end of the fourth side wall away from the first side wall.

5. The biological particle sorting channel according to claim 4, **characterized by** further comprising a plurality of fourth channel units and a plurality of fifth channel units, wherein the radius of curvature of the fourth channel unit is less than that of the fifth channel unit, the radius of curvature of the third channel unit is greater than that of the fifth channel unit, the fourth channel unit and the fifth channel unit are disposed alternately, and the deepened channel correspondingly extends into the fourth channel unit and the fifth channel unit; and the third channel unit is rotated by 90° relative to the fifth channel unit, and the fourth channel unit comprises a seventh side wall and an eighth side wall which are disposed oppositely and are asymmetric curved surfaces, and the fifth channel unit comprises a ninth side wall and a tenth side wall which are disposed oppositely and are asymmetric curved surfaces, the eighth side wall being connected to the ninth side wall, the seventh side wall being connected to the tenth side wall, the seventh side wall of the fourth channel unit at the head being connected to the end of the fifth side wall away from the third side wall, the eighth side wall of the fourth channel unit at the head being connected to the end of the sixth side wall away from the fourth side wall, and at least one of the fifth channel units being provided with a flow dividing hole penetrating a wall surface of the fifth channel unit.

6. The biological particle sorting channel according to claim 5, **characterized in that** the fifth channel unit is provided with a blocking member corresponding to an inlet of the flow dividing hole, the blocking member being located on the side of the flow dividing hole away from the deepened channel, and the width of the blocking member in an extension direction of the fifth channel unit being greater than the diameter of the flow dividing hole.

7. The biological particle sorting channel according to any one of claims 5-6, **characterized by** further comprising a sixth channel unit that is in communication with the fourth channel unit at the tail, wherein the radius of curvature of the sixth channel unit is greater than that of the fifth channel unit, the deepened channel correspondingly extends into the sixth channel unit, the sixth channel unit is rotated by 90° relative to the fifth channel unit, the sixth channel unit protrudes in the opposite direction of the third channel unit, and the sixth channel unit comprises an eleventh side wall and a twelfth side wall disposed oppositely, the eleventh side wall and the twelfth side wall being asymmetric curved surfaces, the eleventh side wall being connected to the end of the eighth side wall away from the ninth side wall, and the twelfth side wall being connected to the end of the seventh side wall away from the tenth side wall.

8. The biological particle sorting channel according to claim 7, **characterized in that** the deepened channel has a depth of 50 µm to 200 µm; or the deepened channel has a depth of 70 µm to 120 µm.

9. The biological particle sorting channel according to claim 7, **characterized in that** the radius of curvature of the first side wall is greater than or less than that of the second side wall, and the first side wall and the second side wall protrude toward the same side;
and/or, the radius of curvature of the third side wall is less than that of the fourth side wall, and the third side wall and the fourth side wall protrude toward the same side;
and/or, the radius of curvature of the fifth side wall is less than that of the sixth side wall, and the fifth side wall and the sixth side wall protrude toward the same side;
and/or, the radius of curvature of the seventh side wall is less than that of the eighth side wall, and the seventh side wall and the eighth side wall protrude toward the same side;
and/or, the radius of curvature of the ninth side wall is less than that of the tenth side wall, and the ninth side wall and the tenth side wall protrude toward the same side;
and/or, the radius of curvature of the eleventh side wall is less than that of the twelfth side wall, and the eleventh side wall and the twelfth side wall protrude toward the same side.

10. A microfluidic chip, **characterized by** comprising a functional board, wherein a first side surface of the functional board is provided with the biological particle sorting channel according to any one of claims 1-9.

11. A microfluidic chip, **characterized by** comprising a functional board, wherein a first side surface of the functional board is provided with the biological particle sorting channel according to any one of claims 5-9, and a second side surface of the functional board is provided with a buffer channel of a repeatedly folded structure, the buffer channel being in communication with the flow dividing hole.
